# EUROPEAN PATENT SPECIFICATION

(11) **EP 1 450 835 B1**
(45) Date of publication and mention of the grant of the patent: **20.09.2006**
(21) Application number: 02804752.0
(22) Date of filing: 09.12.2002
(51) Int. Cl.: A61K 36/00, A61P 29/00

(54) **COMPOSITIONS FOR THE TREATMENT OF LUPUS**
ZUBEREITUNGEN ZUR BEHANDLUNG VON LUPUS
COMPOSITIONS DESTINEES AU TRAITEMENT DU LUPUS

(30) Priority: 07.12.2001 US 339199 P
(43) Date of publication of application: 01.09.2004
(73) Proprietor: Ziegler, Randy H., Newport Coast, CA 92657 (US)
(72) Inventor: Ziegler, Randy H., Newport Coast, CA 92657 (US)
(74) Representative: Siegert, Georg
(86) International application number: PCT/US2002/039297
(87) International publication number: WO 2003/049725

(56) References cited:
- EP-A- 1 103 272
- WO-A-02/07768
- TARGONI OLEG S ET AL: "Prevention of murine EAE by oral hydrolytic enzyme treatment" JOURNAL OF AUTOIMMUNITY, LONDON, GB, vol. 12, no. 3, May 1999 (1999-05), pages 191-198, XP002181698 ISSN: 0896-8411

## Description

### Background of the Invention

### Area of the Art

The present invention concerns a treatment for Lupus.

### Description of the Prior Art

Lupus Erythematosus ("Lupus") is a chronic inflammatory disease that can affect the skin, joints, blood, and kidneys as well as other parts of the body. Lupus is an "autoimmune" disease in which the immune system makes antibodies directed against parts of the body. Normally antibodies react only with bacteria, viruses and other foreign substances. When "self" antibodies are made, damage can occur either through direct antibody mediated attack on body tissues or indirectly from immune complexes. Immune complexes are the reaction products between portions of the body's tissues and the antibodies. These complexes build up in the skin or in joints or in kidneys and cause many of the symptoms of Lupus. Although many cases of Lupus are mild, the disease may cause serious life-threatening symptoms.

Lupus is one of a number of "rheumatic" diseases that generally also include an autoimmune component. Usually much of the damage of Lupus and other rheumatic diseases is mediated by inflammatory processes. Inflammation is generally a process wherein circulation to a region of tissue is increase and blood vessel walls become leaky so that white blood cells can infiltrate the region and attack foreign bodies. If autoantibodies are involved the bodies own tissues are attacked and damaged or destroyed. If the inflammation occurs in the joints, the cushioning and lubricating cartilage of the joints can be damaged. This causes frictional damage to the bones of the joint, which can result in more inflammation and more damage. This is why anti-inflammatory drugs are often used to treat Lupus and other inflammatory joint diseases. It is generally believed that some joint diseases such as osteoarthritis are more of a "wear and tear" disease wherein the cartilage is naturally eroded. However, the end product is still inflammation (which causes further damage) so that anti-inflammatory drugs are also effective.

The most commonly used anti-inflammatory drugs are non-steroidal anti-inflammatory drugs such as ibuprofen or ketoprofen which act on the inflammatory processes downstream of the white blood cells. Steroids directly affect the white blood cells by slowing their replication or b speeding their destruction through apoptosis. Other effective drugs are cytotoxic drugs such as lmuran which suppress the immune system (and hence inflammation) by damaging rapidly dividing cells such as immune cells.

As will be appreciated, all of these drug treatments can have serous side effects. Non-steroidal anti-inflammatory drugs can cause serious damage to the digestive system and often prolong bleeding times. Steroids can be adductive and have a wide range of serious side effects including diabetes. Cytotoxic drugs can result in oversuppression of the immune system with resulting infections and even cancer. Further, many inflammatory diseases-especially Lupus-may not respond adequately to any of the treatments. Consequently, there remains an extreme need for safe and effective treatments for Lupus and other inflammatory diseases.

### Summary of the Invention

The inventor has found that bioflavonoids, particularly flavonols such as Quercetin, supplemented with Bromelain and Vitamin C, (500 mg of each, in doses three times a day) completely reverse the symptoms of Lupus. Luteolin, Myricetin and Rutin are also effective bioflavonoids in the present invention. Experimental data indicate that Luteolin is somewhat more effective than Quercetin. Luteolin, however, is currently difficult to obtain economically in quantity.

While not wishing to be bound to a single explanation for the working of the present invention, the inventor believes that a significant part of the efficacy is due to the effects of specific bioflavonoids on certain cellular ion channels. In the past it has been shown that abnormal potassium channel expression in certain autoimmune diseases is related to the abnormal immune response. (See "Abnormal K⁺ channel expression in AutoImmune Diseases..." by Chandy and Cahalan published in 1990 by the University of California at Irvine and "Autoimmune diseases linked to abnormal K⁺ channel expression in double-negative CD4⁻CD8⁻T cells," Eur J. Immunol, Cahalan MD, Chandy GK, Grissmer S., 1990, 20: 747-751). It was shown rats with experimental SLE (Systemic Lupus Erythematosus), Diabetes (Type I) and EAE (Experimental Autoimmune Encephalitis) were tested for the Kv1.3 channel, and it was shown found that all three have the CD4⁻ and CD8⁻-T-Cells (double negative T-Cells) expression that inflames the various tissues associated with the respective diseases. All of these diseases show a control of lymphocyte proliferation by the Kv1.3 channel. It has independently been demonstrated that flavonoids, especially Quercetin, are very effective at modulating the conduction by this potassium channel. This suggested to the inventor that flavonoids could be effective in controlling human Lupus.

### Detailed Description of the Invention

The following description is provided to enable any person skilled in the art to make and use the invention and sets forth the best modes contemplated by the inventor of carrying out his invention. Various modifications, however, will remain readily apparent to those skilled in the art, since the general principles of the present invention have been defined herein specifically to provide an effective treatment for Lupus.

Flavonoids are ubiquitous secondary products found in most land plants. Flavonoids are oxygen based rather than nitrogen based like traditional pharmaceuticals plant compounds such as alkaloids. As such they are end terminus electron acceptors rather than electron donors. Flavonoids inhibit 5-lipoxygenase in the cytokine release pathways because hydroxyl groups at 4', 3, and 7 positions of the flavonoid molecules accept electrons. (See, Effects of FLAVONOIDS on Arachidonic Acid Metabolism, A. F. Welton, L.D. Tobias, The Biochemistry of Cell Activation Related to the Putative Action of FLAVONOIDS, S. G. Laychock, Plant Flavonoids in Biology and Medicine: Biochemical, Pharmacological, and Structure-Activity Relationships, pg 231-242, 1986 Alan R Liss, Inc.). Flavonoids modulate the immune response through sequestration of free radicals, which prevents formation of epoxide diols and subsequent attack on the DNA. Further, catalytic ion and signal transducers are sequestered by Flavonoids (See, Structural dependence of flavonoid interactions with Cu2⁺ ions: implications for their antioxidant properties, Brown JE, Khodr H, Hider RC, Rice-Evans CA, Biochem. J. 1998 Mar 15;330 (pt 3): 1173-8).

A number of biological effects of flavonoids have been elucidated. For example, flavonoids inhibit Deiodinase, which is the enzyme that promulgates thyroid functions. lodothyronine deiodinase is oxygen bound and directly impacts basal oxygenation. Flavonoids have the steric binding mimicry of ligands that bind the oxygen transport molecule lodothreonine Deiodinase. ("Role of FLAVONOIDS in the Oxygen-free Radical Modulation of Immune Responses", B. Pignol, et. al., Role of Flavonoids in the Oxygen-Free Radical Modulation of the Immune Response, Plant Flavonoids in Biology and Medicine II: Biochemical, Cellular, and Medicinal Properties, pg. 173-182, 1988 Alan R. Liss, Inc.; "Structure Activity Relationships of FLAVONOIDS Deiodinase Inhibitors and Enzyme Active Site Model", S. V. Cody, Plant Flavonoids in Biology and Medicine: Biochemical, Pharmacological, and Structure-Activity Relationships, 1986 Alan R. Liss, Inc., pg. 373-382). Flavonoids thus "turn down the thermostat" of homeostasis. ("lodothyronine Deiodinase is Inhibited by Plant FLAVONOIDS", J. Koehrle pg. 324, Structure Activity Relationships of FLAVONOIDS Deiodinase Inhibitors and Enzyme Active Site Model, S. V. Cody, Plant Flavonoids in Biology and Medicine: Biochemical, Pharmacological, and Structure-Activity Relationships, 1986 Alan R. Liss, Inc., pg. 373-382).

It is believed that the effect of flavonoids in the present invention is mediated through the above-discussed effect on ion channels. However, the effect on the thyroid indicates that the flavonoids may be acting on more than one biological system in the present invention. The inventor included Bromelain in the present invention because there is some indication that this enzyme can aid in the regeneration of cartilage in a damaged joint. Generally, Bromelain alone is not effective in ameliorating the joint pain in either osteoarthritis or Lupus caused joint inflammation. It is logical that the compound is not effective in Lupus because continued Lupus-induced inflammation would mask any joint repairing effects of Bromelain. It also seems apparent that osteoarthritis, at least in some cases, is mediated by an inflammatory process. This may explain why Bromelain treatment of that disease has not been very effective.

However, the flavonoid component of the present invention controls inflammation through its effects on ion channels and possibly through other biological effects not yet elucidated. White blood cells are deactivated by the changes in ion channels so that inflammatory attacks on the joints are decreased. Down regulation of the immune system (as mediated by white blood cells) results in lowered production of autoantibodies. Under these conditions Bromelain appears to be able to help in joint repair. It does seem likely that Bromelain is in some way synergistic with the flavonoids because treatment with flavonoids but without Bromelain is clearly less effective than the combination. Many natural foods contain flavorioids; however, few, if any, contain Bromelain combined with effective flavonoids. Also, it is not known whether the combination of flavonoids in natural products might decrease the overall effectiveness of the "correct" flavonoids. For whatever reasons mere dietary treatments for Lupus and related diseases has not been successful. While the present inventor believes that the addition of vitamin C to the flavonoid/Bromelain mixture enhances the effect of the mixture, it is apparent that the mixture without vitamin C is also effective. However, because vitamin C is relatively innocuous, it has been used in the vast majority of tests.

Screening tests by experts in ion channel function have indicated that a number of flavonoids, especially certain flavonols have the correct charge structure to regulate the key ion channels. Quercetin, which has been used in the majority of tests of the present invention, is very effective in tests of ion channel function. Luteolin is even more effective than Quercetin in such tests. Limited tests have shown that replacing Quercetin with Luteolin in the inventive formula is somewhat more effective. However, at this time the inventor lacks a ready source of pharmaceutical grade Luteolin to demonstrate whether luteolin is significantly more effective overall. Quercetin and Luteolin differ by a single hydroxyl group (which Luteolin lacks as compared to Quercetin). A limited number of tests have shown that Myricetin is somewhat effective in the present; this compound varies from Quercetin in the addition of one hydroxyl. In addition, rutin is also moderately effective in the present invention. Rutin is a glycoside (rutinoside) of Quercetin so that metabolism of Rutin is likely to make Quercetin available. It is likely that other flavonoids with structural similarities to quercetin or luteolin, or glycosides of these flavonoids, will also function in the present invention.

The results of treating actual patients with the inventive composition have been extremely dramatic. Besides the cases described herein literally dozens of patients have shown similar results when treated with the Quercetin/Bromelain/Vitamin C mixture.

One of the first tested subjects was, BZ, who had been bed ridden for nine months and could neither sit up or turn over. She showed the typical Lupus butterfly rash and was diagnosed by a blood test showing a positive ANA (anti-nucleic acid antibody) titer-criteria indicative of Lupus. The subject's mother had previously tested positive for Lupus and has been treated with several medications, including Plaquinel and Cyclosporin. A second subject, KA, had also tested positive for Lupus and had been being treated with Plaquinel and Cyclosporin.

Both of these test subjects experienced rapid relief from symptoms of fatigue and depression and pain, elimination of the associated rash and a return to normal life style within one to two months of beginning a regime of one pill (500 mg Quercetin/500 mg Bromelain/500 mg Vitamin C) three times per day at the above dosage. Kidney flares ceased and pain abrogated. Both subjects stated that they felt completely fine with no side effects and maintained a perfect state of health while on the inventive composition.

Both BZ and KA stopped taking the medication after two months, since they reported that they were "completely recovered." As might be expected, symptoms begin to reappear within two weeks. Upon reestablishment of treatment all symptoms again disappeared. This indicates that the inventive product is solely responsible for the amelioration of SLE (Systemic Lupus Erythematosus).

Additional patients have also shown similar positive results. MK was a 65-year-old woman with seropositive rheumatoid arthritis. She developed side effects from methotrexate treatment at a dose of 22.5 mg weekly. Her methotrexate was discontinued and she was started on the inventive composition. Within one month she showed a dramatic decrease in synovitis.

NM was a 37-year-old woman with an inflammatory polyarthritis and positive ANA (antinuclear antibody). She had Sjogrens syndrome with a positive SSA (anti-Ro) antibody. After three months of treatment with the inventive composition, she noticed decreased hair loss, and a lessening of joint pain and swellings. Her serologies also improved. Her initial ANA titer was 170 and SSA titer was 584, and occasionally much, much higher. Her most recent serologies show an ANA titer of 67 and SSA of 429.

DS was a 57 year old female with SLE/Sjogrens. Her disease manifestations were pulmonary infiltrates and pulmonary effusions. Despite high doses of prednisone, plaquenil, and Imuran she had continued pulmonary symptoms, fevers, joint swellings, and elevated anti-DNA antibody. After one-month treatment with the inventive composition, her Anti-DNA level decreased from 454 to 186 (normal less than 30).

LC was a 46 year old female with SLE. Her main manifestations have been joint inflammation and recurrent serositis. She has had recurrent chest pain and palpitations. Her echocardiogram in 1999 showed valvular changes with trace mitral regurgitation and mild to moderate tricuspid regurgitation. She also had pulmonary hypertension with a RV systolic pressure of 45. LC started the inventive compositions and after four months was tapered off prednisone of 20-mg qd and Plaquinel. Her recent echocardiogram shows improvement-there was no longer any evidence of pulmonary hypertension.

At this time more than 50 patients, most with some form of Lupus, but others with various inflammatory joint diseases, have been treated with the inventive composition. Virtually all patients have demonstrated a measurable amelioration of their disease state.

## Claims

1. Use of flavonoid and Bromelain for the preparation of a pharmaceutical composition effective in the treatment of Lupus.

2. Use according to claim 1, wherein the composition further comprises Vitamin C.

3. Use according to claim 1, wherein the flavonoid is selected from the group consisting of Luteolin, Quercetin, Myricetin and Rutin.

4. Use according to claim 1, wherein the flavonoid comprises a flavonol.

5. Use according to claim 1, wherein the flavonoid comprises a glycoside.

6. Use according to claim 5, wherein the glycoside has an aglycone that is a flavonol.

7. Use according to claim 6, wherein the flavonol is selected from the group consisting of Quercetin, Luteolin and Myricetin.

8. Use according to claim 1, wherein the flavonoid is selected to interact with lymphocyte Kv1.3 channels.

9. Use according to claim 6, wherein the flavonol interacts with lymphocyte Kv1.3 channels.

10. Use according to claim 2, wherein the flavonoid comprises Quercetin.

11. Use according to claim 1, wherein the pharmaceutical composition is for repeated administration.

12. Use according to claim 1, wherein the pharmaceutical composition is for oral administration.

13. Use according to claim 1, wherein the pharmaceutical composition is adapted for repeated administration at least daily.

14. Use according to claim 1, wherein the pharmaceutical composition is adapted for repeated administration at least twice daily.

## Patentansprüche

1. Verwendung von Flavonoid und Bromelain zur Herstellung einer für die Behandlung von Lupus wirksamen pharmazeutischen Zusammensetzung.

2. Verwendung gemäß Anspruch 1, wobei die Zusammensetzung ferner Vitamin C umfasst.

3. Verwendung gemäß Anspruch 1, wobei das Flavonoid aus der Gruppe bestehend aus Luteolin, Quercetin, Myricetin und Rutin ausgewählt ist.

4. Verwendung gemäß Anspruch 1, wobei das Flavonoid ein Flavonol umfasst.

5. Verwendung gemäß Anspruch 1, wobei das Flavonoid ein Glycosid umfasst.

6. Verwendung gemäß Anspruch 5, wobei das Glycosid ein Aglycon besitzt, das ein Flavonol ist.

7. Verwendung gemäß Anspruch 6, wobei das Flavonol aus der Gruppe bestehend aus Quercetin, Luteolin und Myricetin ausgewählt ist.

8. Verwendung gemäß Anspruch 1, wobei das Flavonoid so ausgewählt ist, dass es mit Lymphozyt Kv1.3-Kanälen wechselwirkt.

9. Verwendung gemäß Anspruch 6, wobei das Flavonol mit Lymphozyt Kv1.3-Kanälen wechselwirkt.

10. Verwendung gemäß Anspruch 2, wobei das Flavonoid Quercetin umfasst.

11. Verwendung gemäß Anspruch 1, wobei die pharmazeutische Zusammensetzung eine zur wiederholten Verabreichung ist.

12. Verwendung gemäß Anspruch 1, wobei die pharmazeutische Zusammensetzung eine zur oralen Verabreichung ist.

13. Verwendung gemäß Anspruch 1, wobei die pharmazeutische Zusammensetzung zur wiederholten Verabreichung zumindest täglich angepasst ist.

14. Verwendung gemäß Anspruch 1, wobei die pharmazeutische Zusammensetzung zur wiederholten Verabreichung zumindest zweimal täglich angepasst ist.

## Revendications

1. Utilisation de flavonoïde et de broméline pour la préparation d'une composition pharmaceutique efficace dans le traitement du lupus.

2. Utilisation selon la revendication 1, dans laquelle la composition comprend en outre de la vitamine C.

3. Utilisation selon la revendication 1, dans laquelle le flavonoïde est choisi dans le groupe constitué de lutéoline, quercétine, myricétine et rutine.

4. Utilisation selon la revendication 1, dans laquelle le flavonoïde comprend un flavonol.

5. Utilisation selon la revendication 1, dans laquelle le flavonoïde comprend un glycoside.

6. Utilisation selon la revendication 5, dans laquelle le glycoside possède un aglycone qui est un flavonol.

7. Utilisation selon la revendication 6, dans laquelle le flavonol est choisi dans le groupe constitué de quercétine, lutéoline et myricétine.

8. Utilisation selon la revendication 1, dans laquelle le flavonoïde est choisi de sorte à interagir avec les canaux Kv1.3 des lymphocytes.

9. Utilisation selon la revendication 6, dans laquelle le flavonol interagit avec les canaux Kvl.3 des lymphocytes.

10. Utilisation selon la revendication 2, dans laquelle le flavonoïde comprend la quercétine.

11. Utilisation selon la revendication 1, dans laquelle la composition pharmaceutique est destinée à une administration répétée.

12. Utilisation selon la revendication 1, dans laquelle la composition pharmaceutique est destinée à une administration orale.

13. Utilisation selon la revendication 1, dans laquelle la composition pharmaceutique est adaptée pour une administration répétée au moins quotidienne.

14. Utilisation selon la revendication 1, dans laquelle la composition pharmaceutique est adaptée pour une administration répétée au moins deux fois par jour.
